# EUROPEAN PATENT SPECIFICATION

(11) **EP 2 772 539 B1**
(45) Date of publication and mention of the grant of the patent: **20.06.2018**
(21) Application number: 13000989.7
(22) Date of filing: 27.02.2013
(51) Int. Cl.: C12N 15/70, C12N 15/79

(54) **BARI PROMOTER SEQUENCES AND ITS USES**
BARI-PROMOTORSEQUENZEN UND IHRE VERWENDUNGEN
SÉQUENCES DE PROMOTEUR BARI ET SES UTILISATIONS

(43) Date of publication of application: 03.09.2014
(73) Proprietor: Universita' degli Studi di Bari, 70121 Bari (IT)
(72) Inventor: CAIZZI, Ruggiero Vincenzo, 70125 Bari (BA) (IT); MARSANO, Rene' Massimiliano, 70125 Bari (BA) (IT); PALAZZO, Antonio, 70125 Bari (BA) (IT)
(74) Representative: Trupiano, Federica

(56) References cited:
- CAIZZI RUGGIERO ET AL: "Bari-1, a new transposon-like family in Drosophila melanogaster with a unique heterochromatic organization", GENETICS, vol. 133, no. 2, 1993, pages 335-345, XP002706462, ISSN: 0016-6731 -& DATABASE EMBL [Online] 24 September 1992 (1992-09-24), "D.melanogaster Bari-1 mobile element DNA", XP002706463, retrieved from EBI accession no. EM_STD:X67681 Database accession no. X67681
- ROBERTA MOSCHETTI ET AL: "Conserved motifs and dynamic aspects of the terminal inverted repeat organization within Bari-like transposons", MOLECULAR GENETICS AND GENOMICS, SPRINGER, BERLIN, DE, vol. 279, no. 5, 5 February 2008 (2008-02-05), pages 451-461, XP019630973, ISSN: 1617-4623
- MOSCHETTI R ET AL: "Intra- and interspecies variation among Bari-1 elements of the melanogaster species group", GENETICS 199809 US, vol. 150, no. 1, September 1998 (1998-09), pages 239-250, XP007922109, ISSN: 0016-6731

### SEQUENCE LISTING

<110> Università degli Studi di Bari
<120> SEQUENZE PROMOTORE BARI E SUOI USI
<130> FT/PV 12830
<160> 6
<170> PatentIn version 3.5
<210> 1
   <211> 377
   <212> DNA
   <213> artificial sequence
<220>
   <223> 5' TIR nuclotide sequence for its use as promoter derived from Bari1 of D.melanogaster (Acc.N.X67681)
<400> 1
   cagtcatggt caaaattatt ttcacaaagt gcatttttgt gcatgggtca caaacagttg      60
   cttgtgcagc aagtgggggg aggtgaaatg caaaaaaact tttgcttttg caaattcaaa     120
   cctatgcaga gtcagatgaa agaagaattg aaaaaataac tgttcctatg cgcaaggaag     180
   aggcaaatga agagatcttt atcagttgtc agaagtattt gcacacggtt tcgtcgcatc     240
   acaattattt tcacaacgca atttcttctt cagtgattgg tttagagtga caagtgccgg     300
   tttgtttgct taaatacatt taaattattg aataaaaatt agatttaatc attttcctat     360
   tacagttatt aaataaa                                                    377
<210> 2
   <211> 356
   <212> DNA
   <213> artificial sequence
<220>
   <223> 5'TIR nucleotide sequence for its use as promoter derived from Bari3 of D. mojavensis (Acc. No. CH933806)
<220>
   <221> promoter
   <222> (1)..(356)
<400> 2
   cagaggtggt caaaagtaat tacacaccga gcttttttct gcatgctcag ctttaaaagc      60
   ttgttgttgt tgttgttgtt cgctgttttt gaaaaaaagc ttttgtgtca taaagcttat     120
   tctgtgtaga agcgatcggc aaaaagaaat tgctaaaata acggtacagg ttcgtagata     180
   tagcaaaaag aacagttacc tagaggttgt caaaagtatt tacacacatt ttttttgcgt     240
   cataagtatt tacacaatgt aaatttgcct ttagtctttg atttgcgttg atcatgtgcg     300
   gtctttgact aagatcggaa ttcaatttgt agttgaaatt gtagatgtgc tgtaaa         356
<210> 3
   <211> 30
   <212> DNA
   <213> artificial sequence
<220>
   <223> Primer forward
<400> 3
   gatactcgag cagtcatggt caaaattatt 30
<210> 4
   <211> 30
   <212> DNA
   <213> artificial sequence
<220>
   <223> primer reverse
<400> 4
   gataccatgg tttatttaat aactgtaata 30
<210> 5
   <211> 29
   <212> DNA
   <213> artificial sequence
<220>
   <223> Primer forward
<400> 5
   gatactcgag cagaggtggt caaaagtaa 29
<210> 6
   <211> 30
   <212> DNA
   <213> artificial sequence
<220>
   <223> primer reverse
<400> 6
   gataccatgg tttacagcac atctacaatt 30

## Description

### ABSTRACT

The invention refers to 5'TIR nucleotide sequences derived from the "Bari" family of transposable elements for the use as promoter in the expression of a transgene in a recombinant genetic construct adapted to be transfected in a host cell system.

### STATE OF THE ART

The present invention refers to the biotechnology field and in particular to nucleotide sequences used as promoters with a high transcriptional activity in different host cell systems.

The production of proteins by means of genetically engineered systems became of fundamental importance for therapeutic, medical and pharmaceutical purposes. Development and progress of production technologies and pharmaceutical knowledges support the production of (human) proteins as a therapeutical option for a large number of diseases. The therapeutical proteins, in fact, provide an essential therapy for the treatment of diabetes (insulin), for terminal kidney diseases (erythropoietin), for viral hepatitides (interferon) for the treatment of natural metabolism errors (lysosomal enzymes) and for coagulation diseases (coagulation factors VII, VIII, IX).

Currently, the problem of the choice of the cellular platform requires several expression vectors to be used, and their substantial difference consists in the use of different regulatory elements among which the promoters to assure suitable transcriptional levels in the selected host system.

The family of transposable elements "*Bari*", here in the following also simply named as Bari, has been described and known a long time ago. Bari is a family of mobile genetic elements, called transposons, isolated within the Drosophila genus belonging to the class of Tc1/mariner transposable elements. The distribution of Bari elements in the genomes of different species of Drosophila is known, as well as the structure of three different sub-families of Bari elements (Bari-1, Bari-2, Bari-3).

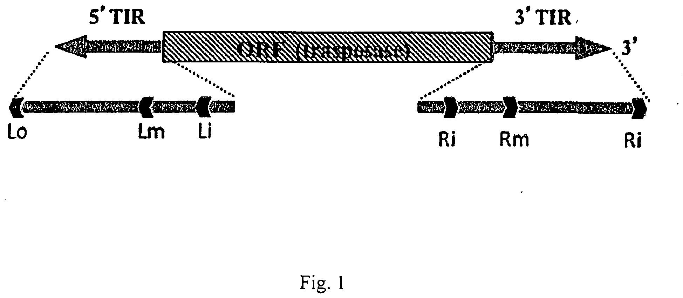

The transposons of the Bari family have a common structural gene organization, as shown in Figure 1, they contain terminal inverted repeats (TIR) sequences at the 5' and 3' ends that delimit the transposase enzyme gene (ORF, "Open Reading Frame"). The length of the TIR region may vary in the nucleotide numerical composition, therefore short (SIR, Short Inverted Repeat) and long (LIR, Long Inverted Repeat) TIR sequences are found. Further studies, (Moschetti et al. Mol Genet Genomics (2008) 279: 451-456) allowed the identification within said TIR of direct repeats (DR) nucleotide sequences with a length comprised between 15-18 nucleotides (nt), said DR representing the recognition sites of the transposase enzyme. Based on the position within the TIR region, the DR sequences, as shown in Fig. 1, may be identified as L (Left) or R (Right), depending if they are on the left or on the right of the ORF region, and with the subscripts "o" (outer), "i" (inner), or "m" (middle), depending if they are localized outside, inside or in the center of the TIR region, respectively.

The known Bari sub-families differentiate for the number of DR sites and the length in the TIR regions.

In the field of molecular manipulation of the desoxyribonucleic acid (DNA) different techniques for the ectopic expression of gene sequences are known. With the term ectopic expression it is meant the forced expression of one or more sequences of interest within cell systems that would not normally express such genes or the expression of such sequences at specific stages of the cell development and differentiation that do not provide for the expression of such gene. In general, the purposes for which ectopic expression studies are carried out vary from the characterization of the functions of still unknown gene sequences to the production of recombinant proteins in heterologous cell systems, that is the protein expression in an organism that would not normally produce them. The known heterologous cell systems are both of prokaryotic origin, usually bacteria, and more complex cell systems of eukaryotic origin.

In order to have the ectopic expression in an heterologous cell systems it is essential that the expression vector is able to contain all signals the host organism needs to transcript the gene, translate it into a polypeptide, realize post-translation modifications and, in case, excrete the protein. Then an expression vector is a nucleic acid molecule allowing the expression of the sequences of interest within the cell/ animal system of choice. Among the signals the expression vector must contain a key element is represented by the promoter. The promoter is a nucleotide sequence that is found upstream the sequence coding for the gene of interest, recognized in a very similar way by the RNA polymerase and allowing the start of the transcription. A promoter used for the expression of heterologous genes must have a high affinity with the RNA polymerase II of the host heterologous cell system and can be inducible, that is, it must have a simple regulation mode of the ON/OFF type.

The promoter performs the same function both in eukaryotic and prokaryotic (bacterial) genomes, but its operation is definitely different in the two genomes. The bacterial promoter has a very simple structure and consists of at least three and essential recognition sequences for the RNA polymerase, at fixed position, a sequence that is found at -10 (with respect to the transcription start site indicated as +1); a sequence at -35 (still with respect to the transcription start site) and the upstream element (UP). The elements at -10 and -35 are recognized by the ς subunit of the RNA polymerase, whereas UP is recognized by the α subunit.

The eukaryotic promoter has a structural organization of its elements which is significantly more complex. But even for the eukaryotic promoter a modular structure can be identified in which various elements can be identified, for example the CAAT box and the TATA box sequences. The CAAT box is generally found at the position -80 with respect to the transcription start site, and is the modulus conferring specificity and strength on the promoter, whereas the TATA box, found at -25 from the transcription site, is responsible for the formation of the complex of the transcription start.

The eukaryotic and prokaryotic promoters are significantly different with respect to the structure and the organization of the recognition sequences and the use thereof in the expression vectors will be different based on host, prokaryotic or eukaryotic, heterologous cell system.

It is known that no one of these cell systems assures absolute advantages in all situations and for all possible applications. The selection of the cell system for expression will require the identification of a specific expression vector and specifically built to contain the gene of interest. At the moment the problem of the selection of the cell system requires that different expression vectors should be used and "designed" ad hoc based on the cell system where they will be transcripted. That is, the expression vectors are different based on the use of different regulatory elements, among which the promoters, to assure adequate transcription levels in the selected host system. Such a kind of genetic engineering is highly expensive both for the economic aspect and the invested time.

There is the need for finding new expression vectors that will be adapted to be employed in ectopic expression experiments in more than one cell platform.

The present invention goes beyond the limits of the known art and provides a promoter with an unexpected transcriptional activity in prokaryotic and eukaryotic cell systems.

### OBJECTS OF THE INVENTION

Object of the present invention is to provide a promoter with a high transcriptional activity in host cell systems, for example prokaryotic and eukaryotic.

Also an object of the present invention is to provide an expression vector comprising a promoter with a high transcriptional activity and a gene of interest for its expression in host cell systems, for example prokaryotic and eukaryotic.

It is still an object of the present invention to provide a prokaryotic and eukaryotic cell expression system transformed/ transfected with an expression vector that comprises a promoter with a high transcriptional activity.

### DESCRIPTION OF THE FIGURES

Fig. 1 describes the structure of the Bari elements from which the UniBa promoters have been isolated.
Fig. 2 describes a generic plasmidic expression vector containing an UniBa promoter to express the transgene cloned in the multiple cloning site (MCS). In addition an origin of bacterial replication (ORI) for the propagation of the vector and a selectable marker (resistance gene) are represented.
Fig. 3 describes the performances of the UniBal promoter within a cell system of mammal, insect and bacteria.
Fig. 4 compares the performances of UniBal and UniBa3 promoters.
Fig. 5 summarizes the properties of the UniBa promoter with respect to usually employed strong promoters in terms of percentages.

### DESCRIPTION OF THE INVENTION

These and even other purposes and relative advantages that will be better explained in the following description, are obtained from at least one promoter derived from the family of Bari transposable elements with high transcriptional activity in prokaryotic and eukaryotic cell systems.

It is an object of the present invention the use of 5'TIR sequences of the Bari family as a promoter for the activation of the gene transcription in prokaryotic and eukaryotic cell systems.

Indeed it has been unexpectedly discovered that the nucleotide sequences of the 5'TIR region of the Bari family are able to activate the gene transcription, thus being transcriptional promoters.

In particular, a promoter, according to the invention, is any 5' TIR sequence of the family of Bari transposable elements, in which short and direct repeats (DR) sequences are present, indicated as Lo, Lm, Li.

The present invention goes beyond the limits of the known art because the 5' TIR region of the transposable elements of the Bari family from *Drosophila* is used as a promoter for the gene expression in different host cell systems, such as prokaryotic and eukaryotic.

The 5'TIR nucleotide sequences of the Bari family according to the invention have a variable nucleotide length comprised between 28 and 400 bp, more preferably between 356 and 377 bp.

And even the 5'TIR nucleotide sequences of the Bari family according to the invention comprise at least one DR region, preferably two DR regions, and even more preferably three DR regions.

According to a preferred embodiment of the invention the 5'TIR nucleotide sequences of the Bari family have a length of about 360 bp and include three DR regions (Lo, Lm, Li).

The 5'TIR nucleotide sequences derived from the Bari family have been arbitrarily named UniBa (Unique Promoter From Bari Transposons). In the present invention it will be possible to refer to the 5'TIR nucleotide sequences of the Bari family with promoter activity also with the "UniBa" acronym followed by a numeral (e.g., UniBal, UniBa2, UniBa3, etc.).

The Bari-1 subfamily has been identified in the *D. melanogaster* species and is known with the GeneBank accession code (X67681). Bari-1 has terminal SIRs of 26 base pairs (bp) and 3 DRs.

The Bari-2 subfamily (also known as Erl) has been identified in the *D. erecta* species and is known with the GeneBank accession code (Y13853). Bari-2 has terminal LIRs of about 250 base pairs (bp) and 3 DRs.

The Bari-3 subfamily has been identified after the full sequencing of the *D. mojavensis* genome, it has a LIR conserved in different species of Drosophila, *(D. mojavensis, D. pseudoobscura, D. persimilis).* According to a preferred embodiment of the invention the 5'TIR nucleotide sequences for their use as a promoter of gene transcription have been selected from those with SEQ ID Nos. 1 (UniBal) or 2 (UniBa3).

For example the sequence with promoter activity UNIBA1 (SEQ ID 1) has been derived from the Bari1 nucleotide sequence of Bari1, that of UNIBA3 (SEQ ID 2) from Bari3.

It is therefore object of the present invention the use of 5'TIR nucleotide sequences of the Bari family as a promoter for the gene transcription.

The 5'TIR nucleotide sequences used as a promoter are selected from those of the Bari family of the Drosophila genus belonging to the class of Tc1/mariner transposable elements.

It is also an object of the invention the use of the 5'TIR nucleotide sequences of the Bari family, preferably of SEQ ID NOs. 1 or 2, with promoter function for the preparation of expression vectors.

With the term "5'TIR nucleotide sequences derived from the Bari family" it is herein meant all the nucleotide sequences having a similarity percentage equal to at least 70%, preferably 80%, even more preferably 95%, with that of the species of Drosophila belonging to the Drosophila genus.

For example, sequences homologous to the UniBal, derived from the *Bari1* belonging to the *Drosophila melanogaster* species, are considered those with the same activity but from the 5'TIR sequence of the *Drosophila simulans, Drosophila sechellia,* etc. with a similarity percentage equal to at least 70%, preferably 80%, even more preferably 95%, with that of UniBal.

Similarly, for the UniBa3 promoter derived from the Bari3 of *D. mojavensis* (Acc. No. CH933806) it is possible to consider homologous sequences those deriving from *D. pseudoobscura* and *D. persimilis.* etc. with a similarity percentage equal to at least 70%, preferably 80%, even more preferably 95%, with that of UniBa3.

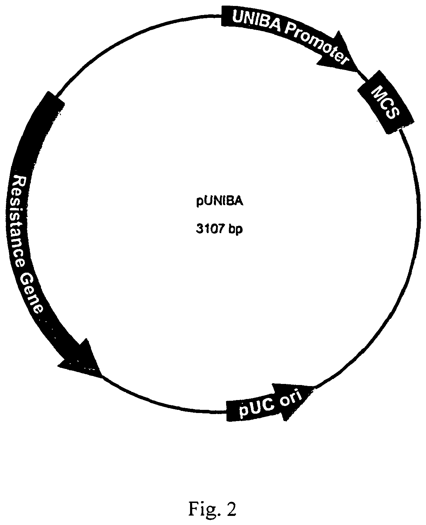

It is still an object of the present invention an expression vector comprising a 5'TIR sequence derived from the Bari family with transcription promoter activity upstream the gene of interest (transgene) to be transcripted, a selection marker that is a gene for resistance to antibiotics, and a replication origin that is a transcription origin site. In Figure 2 a generic plasmidic expression vector according to the invention is represented.

Herein with the term expression vector any "vehicle" for the cloning, the transfer and the expression of a nucleic acid (transgene), that is performed due to the presence of a promoter in a host cell, is meant, for example according to the invention each of plasmidic, cosmidic, phagic, viral and artificial chromosomal vectors are considered expression vectors.

According to a preferred embodiment of the invention the expression vectors comprising the 5'TIR sequences of the Bari family with promoter function are selected from the plasmidic vectors.

The expression vectors according to the present invention goes beyond the limits of the known art because they allow the transcription of the genes of interest both in prokaryotic and eukaryotic cells, thus overcoming the need of using genetically engineered host systems necessary to express the manipulated transgenes. In addition, the expression vectors according to the invention may be easily used for the transfection techniques of any cell line, for example for cultured cell lines for studies of basic research about the gene expression, for the production of recombinant proteins or for toxicological and pharmaceutical studies.

The expression vectors according to the invention, that is vectors in which the 5'TIR nucleotide sequences derived from the Bari family have promoter activity of the gene transcription, may be used for the transfection both in eukaryotic cells and prokaryotic cells without the need of additional factors or of specific genetically engineered cell types, going beyond the limits and complexity of the actually known genetic manipulation.

According to a further preferred embodiment, the invention refers to expression vectors including a nucleotide sequence selected from SEQ ID No.1 and/ or SEQ ID No.2.

Host cell systems, both prokaryotic and eukaryotic, in vitro, ex vivo or in vivo transfected with the expression vectors made according to the present invention, preferably expression vectors including a sequence selected from SEQ ID No.1 and/ or SEQ ID No.2, represent a further object of the present invention.

Herein with the term "host cell systems" is referred to prokaryotic cells such as *Escherichia coli,* eukaryotic cells such as for example insect (S2R+, Sf9 cell) or mammalian (HepG2, HeLa, Hek293, CHO, BHK, human fibrosarcoma cells) cells. According to a preferred embodiment of the invention the host cell systems are selected from Escherichia coli S2R+, Hek293, HepG2.

It is an object of the invention the use of the expression vectors in the so called "basic" or speculative research aimed at identifying the function of new genes and in the industrial field for the production of recombinant proteins (for therapeutic use and not), for gene therapy protocols for the treatment of genetic diseases.

According to a preferred embodiment the present invention refers to the use of expression vectors for the genetic therapy.

It is even an object of the present invention a 5'TIR recombinant isolated nucleotide sequence derived from the Bari family.

According to a preferred embodiment said nucleotide sequence is selected from:
- UNIBA1 (SEQ ID No.1)
- UNIBA3 (SEQ ID No.2)

Therefore the invention has as an object an expression vector comprising at least a 5'TIR sequence derived from the Bari family, said 5'TIR sequence being preferably selected from SEQ ID No.1, SEQ ID No.2.

The present invention refers and has as an object a method to express a sequence of interest, transgene, in a host cell system comprising the steps of:
a) cloning the transgene in an expression vector;
b) transfecting a host cell with an expression vector as defined in the invention;
c) culturing said host cell under proper conditions to obtain the expression of said sequence of interest.

The use of 5'TIR nucleotide sequences derived from the Bari family (for example UniBal or Uniba2) with promoter activity upstream of a transgene of interest, produces several and unexpected advantages allowing the transcription activation in plasmidic and/ or viral expression vectors and their transfection in host cell systems of eukaryotic or prokaryotic origin thus simplifying and optimizing the experimental and the ectopic expression procedures in very different cell systems without the need of performing cloning operations in distinct vectors.

The invention allows to go beyond the limits of the known technique as it provides 5'TIR nucleotide sequences of the Bari family able to operate in any expression vector. Therefore, the sequences with promoter activity according to the invention will allow a new and unexpected approach in the genetic manipulation techniques thus providing a promoter for any expression vector ("one- promoter-for-all-cell-types expression vector").

The proposed invention places itself as a valid alternative to the actually available expression vectors. In the 5'TIR sequences according to the invention, the expression vectors containing the former allow to rapidly switch from experiments and assays in a cell system to another of different origin. The 5'TIR sequences according to the invention will allow to notably speed up the choice procedures of the final platform for the transgene expression (and the respective recombinant protein). Therefore, with the introduction of the expression system based on the use of the UniBari promoters the analysis of the expression of genes or sequences of interest could be carried out in parallel after a single cloning operation for example in three different host cell systems (e.g., mammalian, insect, bacteria). These three systems represent nowadays the principal model systems for studies of basic and applied research.

In this way costs and time for the studies in different cell systems will be significantly reduced. Costs and time for analysis and production of recombinant products at any level (basic research, industrial production of therapeutic proteins etc.) could be decreased by using sequences with promoter activity according to the invention because just one expression vector (recombinant), comprising a 5'TIR sequence derived from the Bari family, will be enough to carry out the analysis and the production of proteins in a plurality of host cell systems.

In addition, the expression vectors according to the invention, as it will be better shown in the following experimental portion, require a promoter to operate. Known promoters are very often "strong", that is promoters activating the gene transcription in a non-adjustable and excessive way and therefore not being able to be used for the study of gene expression or, in the case of protein heterologous expression, they could produce high quantities of recombinant protein thus resulting toxic for the host cell system. The 5'TIR sequences derived from the Bari family with promoter activity go beyond these limits as their transcriptional activity, even not being saturating, guarantees sufficient expression levels in the host cell system.

The present invention is better illustrated by means of the examples reported in the following, in no way being limitative.

### EXAMPLE 1 - Assays of "promoter activity".

In order to verify the efficiency of the 5'TIR sequences of the Bari family as promoters of the transcriptional activity, activities of the UniBal and UniBa3 promoter sequences have been compared with those of known promoters.

Plasmidic vectors have been realized wherein both the UniBal and the UniBa3 sequence have been directionally cloned in the vector commercially available with the name pGL3-basic (Promega Corporation).

The plasmids containing the UniBal and UniBa3 promoter sequences have been tested in two cell lines of human origin (the Hek293 (ATCC) cell line of embryonic origin, and in the HepG2 (ATCC) tumoral line), in a cell line of Drosophila (the line S2R+ (DGRC))), and in a *E.coli* bacterial strain (DH5a (Invitrogen, Life Technologies)). The promoter activity has been measured by means of a luminometric assay of the luciferase enzyme, which represents the used reporter.

In other words, such pGL3-basic vector includes a reporter gene codifying for the luciferase enzyme from the *Photinus pyralis* firefly. The plasmidic vectors have been implemented by using standard cloning procedures in such vector starting from PCR products of the UniBa sequences.

Plasmidic vectors obtained have been tested in order to assess the activity of one of the UniBal or UniBa3 sequences in terms of amount of emitted light.

Data analysis has been carried out by means of transient expression with the plasmidic vectors in two different human cell lines, in an insect cell line, and in a bacterial cell line. The data reported below confirm that both UniBal and UniBa3 behave as promoters in the transcription of all tested cell systems.

Properties of the UniBa sequences have been measured by using an assay based on the activity of the luciferase protein. The luciferase is an enzyme codified by the *luc* gene from *Photinus pyralis* used for the quantitative analysis of elements that potentially regulate both the *cis* (on the same DNA molecule) and *trans* (on a different DNA molecule) gene expression.

The UniBa sequences have been cloned upstream of the luciferase gene, in the pGL3-Basic vector by amplification with specific primers having, at the ends, the sequences recognized by the XhoI and NcoI restriction enzymes. The resulting vector is indicated as pGL3_UniBa. The cloned fragment has been sequenced to verify the correctness of the sequence inserted during cloning.

The pGL3-Basic vector is particularly useful for the purpose of measuring the promoter activity thanks to the presence of a synthetic sequence of polyadenylation upstream of the multiple cloning site, which reduces the transcriptional background due to non-specific sequences.

The plasmidic DNAs have been extracted with commercial purification kits, quantified by reading with a Nanodrop and, later, 1 ug of any single plasmid has been vehiculated in the cells of interest. The transfection methodology is different for eukaryotic and prokaryotic cells. In the first case (eukaryotic cells) the MIRUS TransIt_LT1 (Mirus Bio LLC) transfecting agent has been used as it is specified in the manufacturer manual, whereas in the second case (prokaryotic cells) the calcium chloride method has been used.

Any expression assay has been carried out in triplicate on an adequate cell number, variable depending on the cell type used as an experimental model, but always referring to procedures normally used in homologous assays.

For the definition of the UniBa promoters activity, different control plasmids have been used.
*pGL3B-UniBa1* plasmid object of the test containing the UniBa1 promoter.
*pGL3B-UniBa3* plasmid object of the test containing the UniBa3 promoter.
*pGL3B* provides the expression background for the luciferase (control without promoter).
*pGL3B-copy* provides a reference value in insect cell systems.
*pGL3B-promoter vector* provides a reference value in mammalian cell systems.
*pGL3B-cat* provides a reference value in bacterial cell systems.

The *pGL3-promoter vector* plasmid is commercialized by Promega (catalog number E1761). The SV40 promoter, of viral origin, is widely used as a promoter in expression vectors specific for mammalian lines.

The "copy" promoter found in the *pGL3B-copy* plasmid is of retrotransposon origin and is widely used for the Drosophila expression. It has been amplified starting from the pCoBlast plasmid, commercialized by Life Technologies as part of the DES®-Inducible Kit expression system.

The "cat" promoter found in the *pGL3B-cat* plasmid has been amplified starting from the pHSG396, which contains the full expression cassette for the chloramphenicol-acetyl-transferase.

The cell systems used in order to determine the activity of the UniBal promoter are the following
DH5a™ (Invitrogen, Life Technologies)
Drosophila S2R+ (DGRC) derived from the Schneider's line 2 (DGRC Bloomington, IN, USA)
Human hepatocellular carcinoma HepG2 (ATCC, Manassas, VA, USA)
Hek293 Human Embryonic Kidney 293 cells (ATCC, Manassas, VA, USA).

All the used cell lines are of common use in cell biology and in the basic research. The obtained luminescence results, expressed in RLU (Relative Light Units: RLU = Luminescence/mg of total proteins), have been reported in the following plots, and are relative to measures carried out in plasmids containing the UniBal sequence upstream of the luciferase reporter gene.

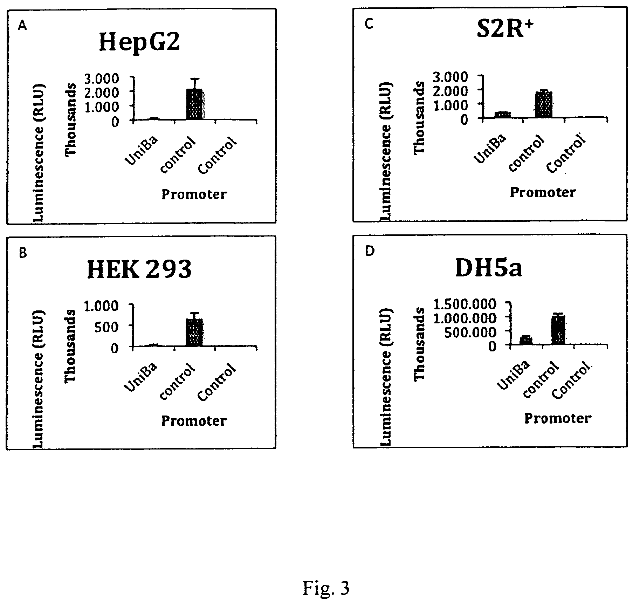

The results are shown in figure 3. Fig. 3A represents the UniBal promoter. UniBal has been tested on human cells and its activity has been compared to that of the SV40 promoter (Simian Virus 40). Negative control is represented by the vector without the sequences activating the transcription.
B: shows that UniBal promoter operates even in a second human cell system with efficiency comparable to that observed in A.
C: shows the UniBal promoter tested in insect cells (S2R+ from Drosophila) and its activity has been compared with that of the Copy promoter derived from the homonymous retrotransposon from Drosophila. Negative control is represented by the vector without the sequences activating the transcription.
D: the UniBal promoter has been tested in bacterial cells (*Escherichia coli* DH5-alpha) and its activity has been compared with that of the cat gene (chloramphenicol acetyl transferase) promoter. Negative control is represented by the vector without the sequences activating the transcription.

Figure 3 demonstrates that UniBal has promoter activity. In particular, in insect eukaryotic and prokaryotic cell systems the measured activity results to be greater, with expression levels around 20% , than those produced by a species-specific promoter.

It is important to point out that all promoters used as positive control for the comparison of activities of the UniBal promoter in different cell systems are universally recognized as very strong promoters (SV40 promoter for mammalian cell systems, copy promoter for insect cell systems, and cat for prokaryotic systems). The same techniques and protocols described above have been used to assay the UniBa3 activity. Therefore these experiments, similar to those shown for the analysis of the UniBal promoter activity, have been carried out for UniBa3. UniBa3 has a nucleotide identity level equal to 18%. UniBa3 derives from the 5'TIR region of the transposable element *Bari3* found in the *D. mojavensis* (Acc. No. CH933806 position 6273693-6275409) genome.

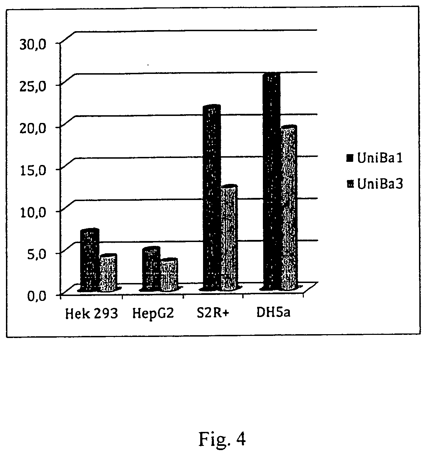

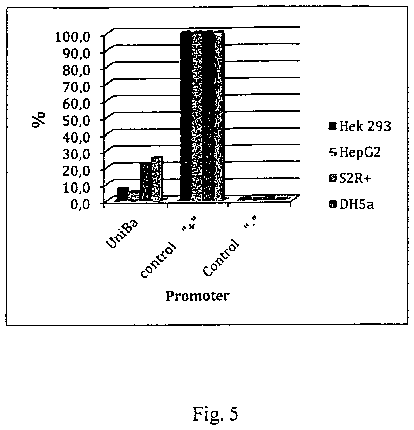

The results obtained for the UniBal and UniBa3 have been compared, as shown in Figure 4. In Figure 5 the same data are presented in terms of percentages.

### EXAMPLE 2: Cloning of the UniBal sequence

5' **TIR UNIBA1** Amplification

### Thermocyclation Parameters

**1 run:**
   Denaturation for 3' at 94°C
**10 runs:**
   Denaturation for 30" at 94°C
   Annealing for 30" at 41°C
   Elongation for 20" at 72°C
**15 runs:**
   Denaturation for 30" at 94°C
   Annealing for 30" at 55°C
   Elongation for 20" at 72°C
**1 run:**
   7' at 72°C
   **4°** ∞

The reaction mixture for the UniBal amplification is composed of:
- Reaction Buffer 1X
- Mg2+ 1.5 mM
- dNTP mix 0.2 mM
- primer TERBari1 up 0.4 µM
- primer TERBari1 low 0.4 µM
- Platinum Taq 1 U
- DNA template 1-5 ng
- Water up to 50 µl

The used enzyme is the Platinum Taq polymerase (Invitrogen, Life Technologies).

Amplified UniBal obtained by using the element present in the 47D region (Ac. No. X67681) (Caizzi et al., Genetics 133 (2), 335-345 (1993)) as template and using the primers
TERBari1 up (forward) gataCTCGAGcagtcatggtcaaaattatt (SEQ ID No. 3)
TERBari1 low (reverse) gataCCATGGtttatttaataactgtaata (SEQ ID No. 4)

The PCR reactions have been carried out in standard conditions by using the Platinum Taq polymerase (Invitrogen, Life Technologies) enzyme.

The TERBari1 up (SEQ ID No. 3) and TERBari3 up (SEQ ID No. 5) oligonucleotides include target sequences for the XhoI restriction enzyme (in capital letters in the "up" primers sequences). The TERBari1 low (SEQ ID No. 4) and TERBari3 low (SEQ ID No. 6) oligonucleotides include target sequences for the Ncol restriction enzyme (in capital letters in the "low" primers sequences). The obtained amplification fragments have been cloned in the XhoI and NcoI sites of the pGL3-Basic (PROMEGA) plasmidic vector, particularly useful for the purpose of measuring the promoter activities thanks to the presence of a polyadenylation synthetic sequence in the multiple cloning site reducing the transcriptional background due to non-specific sequences.

The enzymatic digestion of the amplified UniBal and the pGL3-B vector has been carried out with the following methodology

### Digestion reaction for the PCR samples

- Amplified UniBal 1 µg
- Buffer H (Roche) 1X
- NcoI Enzyme (Roche) 2U
- XhoI Enzyme (Roche) 2U
- Water up to 25 µl
Reactions have been carried out for 2 h at 37 °C

### Digestion reaction for the pGL3-B plasmid

- DNA pGL3-B 1 µg
- Buffer H Roche 10X 1X
- NcoI Enzyme Roche 2U
- XhoI Enzyme Roche 2U
- Water up to 25 µl
Reactions have been carried out for 2 h at 37 °C

### Ligation of the digested fragments

- T4 DNA Ligase Reaction Buffer 1X
- digested pGL3B 50ng
- 5' TIR Unibal 100ng
- T4 DNA ligase (NEB) 1U
- Water up to 20 µl
Reactions carried out at 15°C for about 16 h.

The recombinant plasmidic clones have been purified by commercial kits (QIAGEN plasmid mini kit), quantified by measuring with the Nanodrop and then variable quantities of each single plasmid have been vehiculated in the cells of interest.

### EXAMPLE 3: UniBa3 cloning

Amplified UNIBA3 obtained using as template the "moja" clone (Acc. No. CH933806) (Moschetti et al., 2009) and as primers
TERBari3 up (forward) gataCTCGAGcagaggtggtcaaaagtaa (SEQ ID No. 5)
TERBari3 low (reverse) gataCCATGGtttacagcacatctacaatt (SEQ ID No. 6)

PCR reactions to obtain the amplified UniBa3 have been carried out in standard conditions as reported.

### Thermocyclation Parameters

**1 run:**
   Denaturation for 3' at 94°C
**25 runs:**
   Denaturation for 30" at 94°C
   Annealing for 30" at 55°C
   Elongation for 20" at 72°C
**1 run:**
   7' at 72°C
   4° ∞

The reaction mixture for the UniBa3 amplification is composed of:
- Reaction Buffer 1X
- Mg2+ 1.5 mM
- dNTP mix 0.2 mM
- primer TERBari3 up 0.4 µM
- primer TERBari3 low 0.4 µM
- Platinum Taq 1 U
- DNA template 1-5 ng
- Water up to 50 µl

The used enzyme is the Platinum Taq polymerase (Invitrogen, Life Technologies).

The TERBari1 up and the TERBari3 up oligonucleotides include target sequences for the XhoI restriction enzyme (in capital letters in the "up" primers sequences). The TERBari1 low and the TERBari3 low oligonucleotides include target sequences for the NcoI restriction enzyme (in capital letters in the "low" primers sequences). The obtained amplification fragments have been cloned in the XhoI and NcoI sites of the pGL3-Basic (PROMEGA) plasmidic vector, particularly useful for the purpose of measuring the promoter activities thanks to the presence of a polyadenylation synthetic sequence in the multiple cloning site reducing the transcriptional background due to non-specific sequences.

### Digestion reaction for the PCR samples

- Amplified UniBa3 1 µg
- Buffer H (Roche) 1X
- Ncol Enzyme (Roche) 2U
- XhoI Enzyme (Roche) 2U
- Water up to 25 µl
Reactions have been carried out for 2 h at 37 °C

### Digestion reaction for the pGL3-B plasmid

- DNA pGL3-B 1 µg
- Buffer H (Roche) 10X 1X
- Ncol Enzyme (Roche) 2U
- XhoI Enzyme (Roche) 2U
- Water up to 25 µl
Reactions have been carried out for 2 h at 37 °C

### Ligation of the digested fragments

- T4 DNA Ligase Reaction Buffer 1X
- digested pGL3B 50ng
- 5' TIR Unibal 100ng
- T4 DNA ligase (NEB) 1U
- Water up to 20 µl
Reactions carried out at 15°C for 16 h.

The recombinant plasmidic clones have been selected after E. coli transformation, purified by commercial kits (QIAGEN plasmid mini kit), quantified by measuring at Nanodrop and, later, variable quantities of each single plasmid have been vehiculated in the cells of interest.

### EXAMPLE 4: TRANSFECTION in host cell systems

### TRANSFORMATION IN EUKARYOTIC CELLS

E. Coli cells have been made chemically competent as described in
- Cohen, S.N., Chang, A.C. & Hsu, L. (1972) Nonchromosomal antibiotic resistance in bacteria: genetic transformation of Escherichia coli by R-factor DNA. Proc. Natl. Acad. Sci. USA 69, 2110-2114
- Hanahan, D. (1985) Techniques for transformation of Escherichia coli. In DNA Cloning: a practical approach. Volume 1 (Glover, D.M. ed.), pp. 109-135. Oxford University Press.

The ligation reaction product have been contacted with an aliquot (100 µl) of chemically competent bacteria and have been left for 30 minutes in ice.

Later, a Heat Shock in a thermostatated bath at 42 °C for 60 seconds is carried out and immediately placed in ice for other 60 seconds.

To the transformed bacteria 850 µl of a LB culture medium have been added and they have been left to proliferate at 37 °C for 60 minutes.

At this point the seeding on LB agar plates containing 100 µg/ml of ampicillin is carried out.

The transfection of eukaryotic cells both from Drosophila (S2R+) and human (HepG2 and HEK293), has been realized using the MIRUS TransIt_LTl (Mirus Bio LLC) transfecting agent according to the recommendations the supplier provided about cellular density and DNA quantity to be transfected. Shortly, the used procedure is the following.

### Cells seeding:

10⁵ cell/ cm² for S2R+ cells
Confluence 70% for the Hek293 and HepG2 cells

After 24 h the **transfection** is carried out according to the following protocol:

### Preparation of the mixture

250 µl of growing medium serum free
3 µl Transit - LT1
1 µg plasmid DNA of interest
Incubate 20 minutes at room temperature
Apply dropwise the mixture to the cells.

Control the expression after 48 h
UNIBA1 (SEQ ID No.1)
>UNIBA3 (SEQ ID No.2)
   Primer forward TERBari1 up gataCTCGAGcagtcatggtcaaaattatt (SEQ ID No. 3)
   Primer reverse TERBari1 low gataCCATGGtttatttaataactgtaata (SEQ ID No. 4)
   Primer forward: TERBari3 up gataCTCGAGcagaggtggtcaaaagtaa (SEQ ID No. 5)
   Primer reverse TERBari3 low gataCCATGGtttacagcacatctacaatt (SEQ ID No. 6)

## Claims

1. Use of at least one 5' terminal inverted repeats (TIR) sequence selected from SEQ ID No. 1 (UniBa1), and SEQ ID. No2 (UniBa3), of the Bari family of the Drosophila genus, belonging to the class of Tc1/mariner transposable elements, as promoter for the activation of the gene transcription in prokaryotic and eukaryotic cell systems.

2. Use of at least one 5'TIR sequence according to claim 1, wherein said 5'TIR sequence comprises at least one direct repeat (DR) sequence selected from Lo, Lm, Li.

3. Use of at least one 5'TIR sequence according to claims 1-2, wherein said 5'TIR sequence has a nucleotide length comprised between 356 and 377 bp.

4. Use of at least one 5'TIR sequence according to claims 1-3, wherein said 5'TIR sequence includes at least one DR region.

5. Use of at least one 5'TIR sequence according to claims 1-4, wherein said 5'TIR sequence has a length of about 360 bp and includes three DR regions, Lo, Lm, Li.

6. Use of at least one 5'TIR sequence according to claims 1-5, for the preparation of expression vectors.

7. Use of at least one 5'TIR sequence according to claims 1-6, wherein said 5'TIR sequence is selected from SEQ ID No. 1, SEQ ID NO. 2.

8. Expression vector having promoter activity of the gene transcription in prokaryotic and eukaryotic cells, wherein the promoter includes at least one 5' terminal inverted repeats (TIR) sequence selected from SEQ ID No.1, and SEQ ID No.2, of the Bari family of the Drosophila genus, belonging to the class of Tc1/mariner transposable elements.

9. Method for the expression of a sequence of interest, transgene, in a host cell system comprising the steps of:
a) cloning the transgene in an expression vector as defined in claim 8;
b) transfecting a host cell with said expression vector;
c) culturing said host cell under proper conditions to obtain the expression of said sequence of interest.

## Patentansprüche

1. Verwendung mindestens einer 5'-invertierten terminalen Wiederholungs(TIR)-Sequenz, die aus SEQ ID Nr. 1 (UniBal) oder SEQ ID Nr. 2 (UniBa3) ausgewählt ist, der Bari-Familie der Drosophila-Gattung, zugehörig der Klasse von Tc1/mariner-Transposons als Promotor für die Aktivierung der Gentranskription in prokaryotischen und eukaryotischen Zellsystemen.

2. Verwendung von mindestens einer 5'-TIR-Sequenz gemäß Anspruch 1, wobei die 5'-TIR-Sequenz mindestens eine direkte Wiederholungssequenz (DR-Sequenz) umfasst, die aus Lo, Lm, Li ausgewählt ist.

3. Verwendung von mindestens einer 5'-TIR-Sequenz gemäß einem der Ansprüche 1 bis 2, wobei die 5'-TIR-Sequenz eine Nukleotidlänge zwischen 356 und 377 Basenpaaren aufweist.

4. Verwendung von mindestens einer 5'-TIR-Sequenz gemäß einem der Ansprüche 1 bis 3, wobei die 5'-TIR-Sequenz mindestens eine DR-Region umfasst.

5. Verwendung von mindestens einer 5'-TIR-Sequenz gemäß einem der Ansprüche 1 bis 4, wobei die 5'-TIR-Sequenz eine Länge von ungefähr 360 Basenpaaren aufweist und drei DR-Regionen, Lo, Lm, Li, umfasst.

6. Verwendung von mindestens einer 5'-TIR-Sequenz Sequenz gemäß einem der Ansprüche 1 bis 5 zur Herstellung von Expressionsvektoren.

7. Verwendung von mindestens einer 5'-TIR-Sequenz gemäß einem der Ansprüche 1 bis 6, wobei die 5'-TIR-Sequenz aus SEQ ID Nr. 1 oder SEQ ID Nr. 2 ausgewählt ist.

8. Expressionsvektor mit Promotoraktivität der Gentranskription in prokaryotischen und eukaryotischen Zellen, wobei der Promotor mindestens eine 5'-invertierte terminale Wiederholungs(TIR)-Sequenz umfasst, die aus SEQ ID Nr. 1 oder SEQ ID Nr. 2 ausgewählt ist, einschließlich mindestens einer 5'-TIR-Sequenz, der Bari-Familie der Drosophila-Gattung, zugehörig der Klasse von Tc1/mariner-Transposons.

9. Verfahren zur Expression einer Sequenz von Interesse, transgen, in einem Wirtszellsystem, umfassend die Schritte:
a) Klonieren des Transgens in einen Expressionsvektor wie in Anspruch 8 definiert;
b) Transfizieren einer Wirtszelle mit dem Expressionsvektor;
c) Kultivieren der Wirtszelle unter geeigneten Bedingungen, um die Expression der Sequenz von Interesse zu erhalten.

## Revendications

1. Utilisation d'au moins une séquence de répétitions terminales inversées (TIR) en 5' choisie parmi SEQ ID NO : 1 (UniBal) et SEQ ID NO : 2 (UniBa3), de la famille Bari du genre Drosophila, appartenant à la classe des éléments transposables Tel/mariner, en tant que promoteur pour l'activation de la transcription génique dans des systèmes cellulaires procaryotes et eucaryotes.

2. Utilisation d'au moins une séquence TIR en 5' selon la revendication 1, dans laquelle ladite séquence TIR en 5' comprend au moins une séquence de répétitions directes (DR) choisie parmi Lo, Lm, Li.

3. Utilisation d'au moins une séquence TIR en 5' selon les revendications 1 ou 2, dans laquelle ladite séquence TIR en 5' a une longueur en nucléotides comprise entre 356 et 377 pb.

4. Utilisation d'au moins une séquence TIR en 5' selon les revendications 1 à 3, dans laquelle ladite séquence TIR en 5' comprend au moins une région DR.

5. Utilisation d'au moins une séquence TIR en 5' selon les revendications 1 à 4, dans laquelle ladite séquence TIR en 5' a une longueur d'environ 360 pb et comprend trois régions DR, Lo, Lm, Li.

6. Utilisation d'au moins une séquence TIR en 5' selon les revendications 1 à 5, pour la préparation de vecteurs d'expression.

7. Utilisation d'au moins une séquence TIR en 5' selon les revendications 1 à 6, dans laquelle ladite séquence TIR en 5' est choisie parmi SEQ ID NO : 1, SEQ ID NO : 2.

8. Vecteur d'expression ayant une activité de promoteur de la transcription génique dans des cellules procaryotes et eucaryotes, dans lequel le promoteur comprend au moins une séquence de répétitions terminales inversées (TIR) en 5' choisie parmi SEQ ID NO : 1 et SEQ ID NO : 2, de la famille Bari du genre Drosophila, appartenant à la classe des éléments transposables Tc1/mariner.

9. Procédé pour l'expression d'une séquence d'intérêt, un transgène, dans un système de cellule hôte comprenant les étapes de :
a) clonage du transgène dans un vecteur d'expression tel que défini dans la revendication 8 ;
b) transfection d'une cellule hôte avec ledit vecteur d'expression ;
c) culture de ladite cellule hôte dans des conditions correctes pour obtenir l'expression de ladite séquence d'intérêt.
